# EUROPEAN PATENT APPLICATION

(11) **EP 1 417 933 A1**
(43) Date of publication of application: **12.05.2004**
(21) Application number: 02025172.4
(22) Date of filing: 11.11.2002
(51) Int. Cl.: A61B 17/00, A61B 17/12

(54) **Transcatheter left atrial appendage occlusion prosthesis**

(71) Applicant: Callegari, Sergio, 43036 Fidenza (PR) (IT); Onorato, Eustaquio, 25064 Gussago (BS) (IT)
(72) Inventor: Callegari, Sergio, 43036 Fidenza (PR) (IT); Onorato, Eustaquio, 25064 Gussago (BS) (IT)
(74) Representative: Faraggiana, Vittorio, Dr. Ing.

(57) **Abstract**

A collapsible and implantable device is designed to anchor and occlude the left atrial appendage of the heart. The device comprises an element (10) with a structure made of metallic wires (11) that is elastic pliable and which is expansible from an extended axial contracted condition, in order to pass through a positioning catheter, to an operative expanded condition which has generically disk-shaped form. In the expanded position the device defines seats (13) which are outdistanced on a circumference at 120° from each other on its surface. Each seat (13) has an elastic pliable edge intended to accept and hold in a firm position an area of the atrial appendage which is pushed in it. The seats are advantageously formed by spiral-shape wires. Each seat may consist of several spiral shape wires.

## Description

This invention refers to a device or prosthesis intended to seal the orifice of the left atrial appendage.

It is known that patients suffering from Atrial Fibrillation have a high probability to produce thrombi in the left atrial appendage. Surgical occlusion of the left atrial appendage has been proposed to prevent arterial emboli complications.

The configuration of the left atrial appendage makes it impossible to use expandable prostheses to occlude it, because of the shape of the extroversion which does not allow a firm anchorage of this prosthesis, e.g. of the type disclosed in the PCT patent application WO 99/12478.

The general object of the present invention is to provide a mechanical implantable device designed to occlude the appendage in a firm and safe manner and easy to position with less invasivity.

In view of this object, it is sought to provide in accordance with the present invention a collapsible and implantable device designed to anchor and occlude the left atrial appendage of the heart, comprising an element with a structure made of metal wires that is elastic and pliable and which is expansible from an extended axial contracted condition, in order to pass through a positioning catheter, to an operative expanded condition which has a generically disk-shaped form, in the expanded position the element defining seats which are outdistanced on a circumference from each other on its surface, each seat having an elastic pliable edge designed to accept and hold in a firm position an area of the atrial appendage which is pushed in it.

To clarify the explanation of the innovative principles of the present invention and its advantages compared with the prior art there is described below with the aid of the annexed drawings a possible exemplifying embodiment applying such principles. In the drawings:
Figure 1 represents a frontal view, in an expanded condition, of an occlusion device in accordance with the present invention;
Figure 2 represents a side schematic view of the device shown in figure 1 in a partially collapsed condition;
Figures 3-5 represent a sequence of steps in the positioning procedure of the device illustrated in figure 1, which is shown in section along the line III-III of the figure 1.

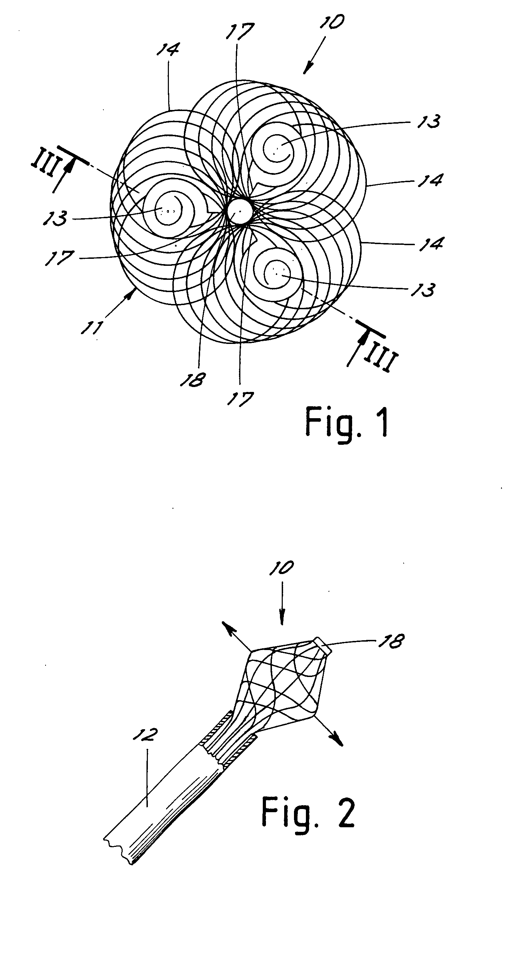

With reference to the figures, figure 1 shows a device or prosthesis, generically indicated with 10, which includes a generically disk-shaped element formed by a structure of elastic metallic wires 11 set in woven loose loops. The wires are made of an elastic deformable metallic material having suitable biocompatible characteristics for implanting. For example, it has been find advantageous to employ wires made of a shape memory alloy in Nickel-Titanium, as the alloy commercially known as Nitinol (55% nickel, 45% titanium).

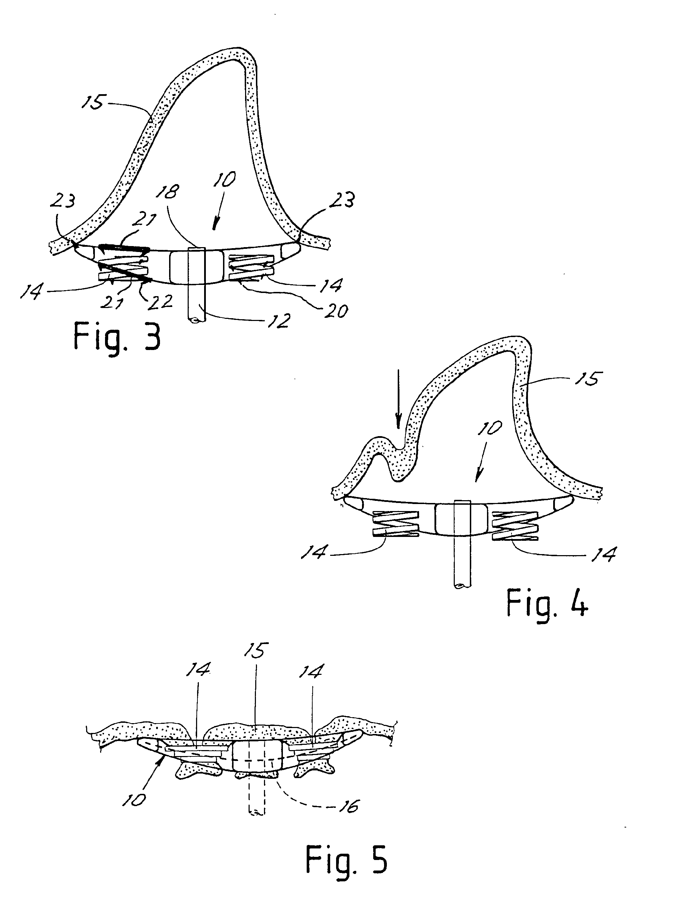

As it is clear also from the comparison between figure 1 and figure 2, the grid of wires is manufactured so that the generically disk-shaped element may be elastically collapsed in a direction parallel to the extension of the disk in order to pass from a contracted, tubular shape, extended along the disk central axis (narrow enough to pass through a catheter 12 to be deployed) to an expanded disk-shaped condition as shown in figure 1 and in section in figure 3.

The generically mesh structure necessary for the elastic expansion which has been mentioned above can be easily imagined by an expert technician and it won't be described further.

As it can be clearly seen in figures 1 and 3, the discoidal element includes on its surface seats or passages 13 with edge pliable in an elastic way to accept and hold the left atrial appendage, as we are going to explain. The seats 13 are preferably three (or fewer), uniformly placed around the central axis at 120° the one from the other. Profitably, each seat is formed at least by a coiled wire 14. The spirals formed by the wires have dimension so as to supply the required elastic strength in a transversal direction to the seat 13, a sufficient strength to keep firmly the atrial appendage. In order to achieve this object multi-start spirals can be used to obtain a sufficiently high radial strength although keeping each elementary wire section thin to favour the high elastic deformability of the structure. Wire 14 may be thicker than the rest of the structure, so that we can obtain the required strength without compromising the capability to compress the structure to make it collapse and to make it pass through the catheter. As clearly shown in figure 1, the coiled wire has an anchorage end 17 which is radially directed towards the centre of the discoidal element where the wires are joined together in a metal pivot 18. As clearly shown in figure 1, the whole structure of wires may have loops which in the plan are disposed in groups around the seats 13 of the atrial appendage insertion.

The surface of the coiled wire can be worked to make it rough or can be worked in order to create on it asperities which can provoke a firmer anchorage of the spiral in the left atrial appendage tissue.

For example, in an advantageous embodiment (see figure 3), grip barbs 20 are formed on the coiled wire 14, in correspondence to the orifice of the passage of the left atrial appendage tissue. Still advantageously barbs 22 are formed on wire segments corresponding to non-pliable edges 21 of the passage for the tissue 15 (two edges 21 are superimposed one upon another in each passage, see on the left of figure 3). The barbs 20, 22 are positioned inclined (advantageously angle < 30°) inwardly the corresponding passage with the points of the barbs directed in the same direction of insertion of the tissue 15 into the passage. Grip barbs 23 can be also advantageously formed on the wires corresponding to the external edge of the device in the expanded condition. The points of the barbs 23 are inclined (advantageously angle < 30°) outwardly the device with the points of the barbs directed in the opposed direction of insertion of the tissue 15 into the device.

In the use, the left atrial appendage occlusion 15 is performed by a trans-septal percutaneous approach so that the prosthesis 10 is positioned against the appendage entrance (figure 3). Afterwards we perform in sequence the introflexion of the atrial appendage wall (e.g. by means of micropliers - not shown- which cross the seats to seize the appendage and subsequently withdraw), so that the wall is firmly inserted inside the seats 13, by pushing against the elastic resistance to expansion of the wire spirals. In order to complete the occlusion the prosthesis may also include a fibre interlacement of material suitable for plugging, advantageously in polyester fibres (e.g. Dacron@) as schematically sketched with 16 in figure 5.

Once the prosthesis is firmly fixed, it can be disconnected from the catheter which can be withdrawn.

At this point it is apparent how we have achieved the intended purposes, supplying an easy implantable prosthesis, with high and stable mechanical characteristics.

Obviously, the above description of an embodiment applying the innovatory principle of the present invention is only given for purposes of illustration of the innovative principles and is not to be interpreted as a limitation of the scope of the patent rights herein claimed.

For example, the proportions between the different parts and their exact configuration may vary according to particular practical needs.

## Claims

1. A collapsible and implantable device designed to anchor and occlude the left atrial appendage of the heart, comprising an element (10) with a structure made of metal wires (11) that is elastic and pliable and which is expansible from an extended axial contracted condition, in order to pass through a positioning catheter, to an operative expanded condition which has a generically disk-shaped form, in the expanded position the element defining seats (13) which are outdistanced on a circumference from each other on its surface, each seat (13) having an elastic pliable edge designed to accept and hold in a firm position an area of the atrial appendage which is pushed in it.

2. Device according to claim 1, **characterized in that** said seats are defined by spiral-shape wires having one or several beginning.

3. Device according to claim 2, **characterized in that** the seat are at least three seats uniformly placed at 120° from each other.

4. Device according to claim 2, **characterized in that** the spiral-shaped wire has an anchorage end (17) which is radially directed towards the centre of the generically disk-shaped element.

5. Device according to claim 1, **characterized in that** the wires are joined together in a central area (18) of the generically disk-shaped element.

6. Device according to claim 1, **characterized in that** the wires structure has loops which in the plan are disposed in groups around said seats.

7. Device according to claim 1, **characterized in that** the metal wires are made of a Nickel-Titanium alloy.

8. Device according to claim 1, **characterized in that** it comprises a plugging wire mesh (16) in polyester fibre, such as Dacron®.

9. Device according to claim 2, **characterized in that** first grip barbs (20) are formed on the spiral-shape wires, the barbs (20) being positioned inclined inwardly the corresponding seat (13) with the points of the barbs directed in the same direction of insertion of the tissue into the seat.

10. Device according to claim 1, **characterized in that** second grip barbs (22) are formed on wire segments corresponding to edges (21) of the seats, the second grip barbs (20) being positioned inclined inwardly the corresponding seat (13) with the points of the barbs directed in the same direction of insertion of the tissue into the seat.

11. Device according to claim 1, **characterized in that** third grip barbs (23) are formed on the wires corresponding to external edge of the device in the expanded condition, the third grip barbs (20) being positioned inclined outwardly the device with the points of the third barbs directed in the opposed direction of insertion of the tissue into the device.
